# EUROPEAN PATENT SPECIFICATION

(11) **EP 3 166 505 B1**
(45) Date of publication and mention of the grant of the patent: **09.10.2019**
(21) Application number: 15818535.5
(22) Date of filing: 08.07.2015
(51) Int. Cl.: A61B 17/064, A61B 17/068, A61B 17/70, A61B 17/80, A61B 17/16, A61B 17/17, A61B 17/00, A61B 50/30

(54) **BONE IMPLANT WITH ANTI-ROTATION**
KNOCHENIMPLANTAT MIT DREHSCHUTZ
IMPLANT OSSEUX ANTI-ROTATION

(30) Priority: 10.07.2014 US 201462022811 P; 12.08.2014 US 201462036240 P
(43) Date of publication of application: 17.05.2017
(73) Proprietor: Crossroads Extremity Systems, LLC, Memphis TN 38119-5702 (US)
(72) Inventor: COLEMAN, Glen, Cordova, Tennessee 38018 (US); HARTDEGEN, Vernon, Collierville, Tennessee 38017 (US); HOLLIS, Michael, Collierville, Tennessee 38017 (US)
(74) Representative: HGF Limited
(86) International application number: PCT/US2015/039556
(87) International publication number: WO 2016/007626

(56) References cited:
- GB-A- 2 118 474
- US-A- 5 788 698
- US-A1- 2002 035 369
- US-A1- 2006 058 802
- US-A1- 2010 125 301
- US-A1- 2014 014 553
- US-A1- 2014 018 809

## Description

### BACKGROUND OF THE INVENTION

### Field of the Invention

The present invention is in the technical field of medical devices. More particularly, the present invention is in the technical field of bone fixation or arthrodesis or deformity correction. The invention relates to a fixation system for bones of all types utilizing an implant. Such systems are used in osteosynthesis (bone fusion), wherein the implant bridges the fracture generating a force (typically compression) across the bone members. The force is generated by either the properties of the implant, the different configurations of the implant, the surgical technique or placement of the implant or a combination thereof. For example, the implant may have a first configuration when in free-state and a second configuration required for insertion. It may be desirable for optimal implant placement and function to be able to pre-assemble or attach the implant to an inserter to facilitate placement of the implant on or in the bone. Once implanted, it is desirable to prevent or minimize rotation or other movement of the bone segments relative to one another. The implant may be indicated for the various bones of the entire skeleton.

### The Related Art

The present invention seeks to remedy the problems of the prior art. The invention produces a system that allows placement of an implant in its final required position with or without additional manipulation. In addition, the present invention will provide resistance to pullout and/or rotation once implanted. The implant features may be particularly beneficial in a compression implant. The current invention may or may not rely on additional implant positioning once positioned in the bone. Also, the current invention may incorporate other necessary features for delivery of the implant into the bone. US 2010/125301 A1 discloses a surgically implantable device, system, and method of use thereof for the internal compression or distraction of, for example, bone fractures, fusions, and osteotomies. More specifically, the surgical device comprises a bridge with one or more adjustable anti-reversing portions. The adjustable anti-reversing portion(s) may be located in a symmetrical or asymmetrical manner on the bridge between the device's legs. The adjustable anti-reversing portion(s) allow for stronger compression of the bone at a known amount of compression. The adjustable anti-reversing portion(s) can also maintain the device's legs in closer proximity to one another for greater compression or maintain the device's legs apart from one another for distraction. The surgical device can also comprise cannulated or grooved legs.

### SUMMARY OF THE INVENTION

The invention is defined in claim 1 while preferred embodiments are set forth in the dependent claims.

The present invention includes an implant or other bone fastening device. The implant may be a bone staple, bone plate, modular staple, or the like. The implant has elastic properties or other material properties that allow the device to have at least two configurations or configurable to various positions when placed on or in the bone. The free-state or implanted-state of the device provides a force, typically compression, across two or more bone members. An inserter is used to hold the implant or other fastening device in a configuration that is different than the free-state or implanted-state configuration. This first configuration may be useful in placement of the implant onto or into bone members. Fastening device and implant are used interchangeably in this application and are not intended to be limiting in nature.

The present invention may have implant legs that once inserted into bone provide a dynamic compression and prevent pullout and/or rotation of the adjoining bone segment. The implant also has features for releasably engaging an implant inserter. The present invention includes an implant or portion of an implant that is made of an elastic material or a material that allows the implant to have multiple configurations. The ability of the implant to have multiple configurations may be achieved by the material properties that have shape memory or super elastic properties or it may be achieved by manipulation (mechanical, physical, or temperature) of the implant to create a second configuration. Alternatively, but not claimed, the ability of the implant to achieve multiple configurations may be achieved by chemical manipulation or electrical manipulation. The implant is held in one configuration during insertion or removal and returns to or is placed in another configuration in its free-state or implanted-state. The implant has multiple configurations, for example one for inserting into the bone and at least a second configuration for compressing, distracting, controlling spatial orientation or the like of one or more bone segments.

The implant has features for engaging the bone. These features may include bone screws, leg members, pins or other features for attaching the implant to bone. The implant may have one or more bridge members. The implant may have leg members for engaging the bone. The implant may have modular members for engaging bone, such as bone screws or pegs. To those skilled in the art, based on the description of the invention herein, it will be evident that multiple options exist for connecting an implant to bone. The connecting members or features may not necessarily be of the same material as the bridge component. The deformability aspect of the current invention may be in the bridge members), the connecting member(s) or another member(s) of the implant or fixation device. The leg member(s) may be configured to receive members from the inserter to hold the implant in its first configuration or to allow manipulation of the implant to another configuration. The leg members also have features or geometry to prevent relative movement between two adjoining bone segments. For example, these features may include fins, projections, non-circular geometries or the like that may prevent rotation of the leg within each bone segment thereby preventing rotation between the adjoining bone segments.

A "bone fixation device" or implant may include any of a variety of devices that secure an object to a bone, including but not limited to staples, bone plates, modular staples, bone screws, pins, blades, suture anchors, and the like. The terms "inserter," "implant inserter," "insertion device," and "delivery instrument" are used herein interchangeably.

### BRIEF DESCRIPTION OF THE DRAWINGS

Figure 1 is a perspective view of a first embodiment of the present invention.
Figure 2 is a front view of a first embodiment of the present invention further showing the location of section A-A.
Figure 3 is the section view A-A of the leg of the first embodiment shown in Figure 2.
Figure 4 is a front view of a second embodiment of the present invention showing alternate leg geometry and a section B-B.
Figure 5 is the section view B-B of the second embodiment shown in Figure 4.
Figure 6 depicts other possible cross sections for other embodiments of the staple legs.
Figure 7 is a perspective view of a third embodiment of the current invention.
Figure 8 is a perspective view of a fourth embodiment of the current invention.
Figure 9 is a top view of an implant kit that may be provided for inserting the implant of the current invention into bone segments.
Figure 10a shows a section view of two bones with an exemplary embodiment of the current invention partially inserted into the bones.
Figure 10b shows a section view of two bones with an exemplary embodiment of the current invention fully inserted into the bones.

### DETAILED DESCRIPTION OF THE INVENTION

The present invention includes an implant for spanning and/or fixating at least two bone segments. The exemplary embodiments of the current invention are discussed in the description of the figures below. The implant may be of a configuration similar to a bone staple as discussed below. The present invention includes an apparatus or instrument for inserting the device that may be pre-assembled or affixed to the implant. The implant or implants could be held in a particular configuration prior to use that facilitates insertion into the bone segments. The embodiments described herein may be used in connection with any type of inserter or fixation device that is compatible with the description and objectives stated herein, including but not limited to various bone staples, bone plates, etc. that may have more than one implant configuration and may generate a force, typically a compressive force, across bone segments.

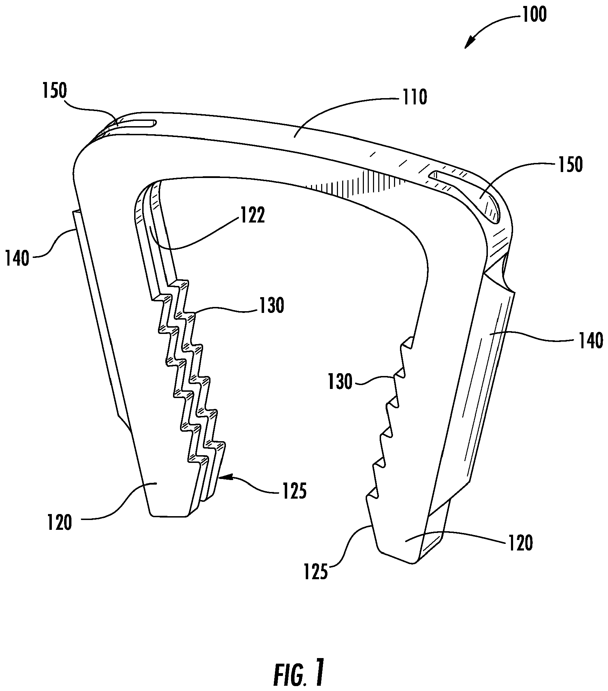

Figure 1 shows a bone implant 100 consisting of a bridge 110 and legs 120. The implant 100 shown in Figure 1 may have legs 120 converging or in a compressed state. This may be the free-state or implanted state of the implant 100. Bridge member 110 may be of uniform cross section or of a varying cross section as depicted in Figure 1. In this particular embodiment bridge member 110 consist of an arched geometry. Legs 120 may have a tapered tip 125 that may facilitate insertion into the bone. Legs 120 may also have barbs 130 that may minimize or prevent pullout and/or rotation. In this exemplary embodiment, legs 120 have barbs 130 on either side of groove 122. This interrupted barb geometry may further resist rotation of the leg 120 in a bone. The barbs 130 are shown on the internal surface of this embodiment, but may also be present on an external surface of the legs 120. The implant 100 has fin like projections 140 to resist rotation of the legs 120 within the bone thereby resisting rotation between the bone segments in which the legs 120 are implanted. In this embodiment the fin 140 is shown on one external surface of the implant leg 120. The fin 140 may also be on more than one surface of leg 120 and not necessarily on the surface directly opposed to the barbs 130. Implant 100 has opening 150 for attachment to an inserter or to allow manufacturing of the non-symmetrical legs 120 and particularly groove 122. As shown in Figure 1, staple legs 120 may have a groove 122 that is defined by the shape of the opening 150 and the outer geometry of the legs 120. The non-circular geometry of the legs 120 when inserted into the bone provides an interference type fit with the bone such that the non-circular geometry will not rotate within the bone. Leg 120 should not rotate within the bone because the non-circular and/or non-symmetrical geometry does not match the prepared hole in the bone. An interference fit within the bone may be generated by using a drill that has a smaller diameter than the circumscribed outer diameter of the implant leg 120 or the prepared hole is of a different geometry than the implant leg 120. Leg 120 may not rotate within the bone because the non-circular and/or non-symmetrical geometry may create a "keyed" interlock with the bone in which the leg is inserted. This keyed geometry may be the negative of the implant leg geometry.

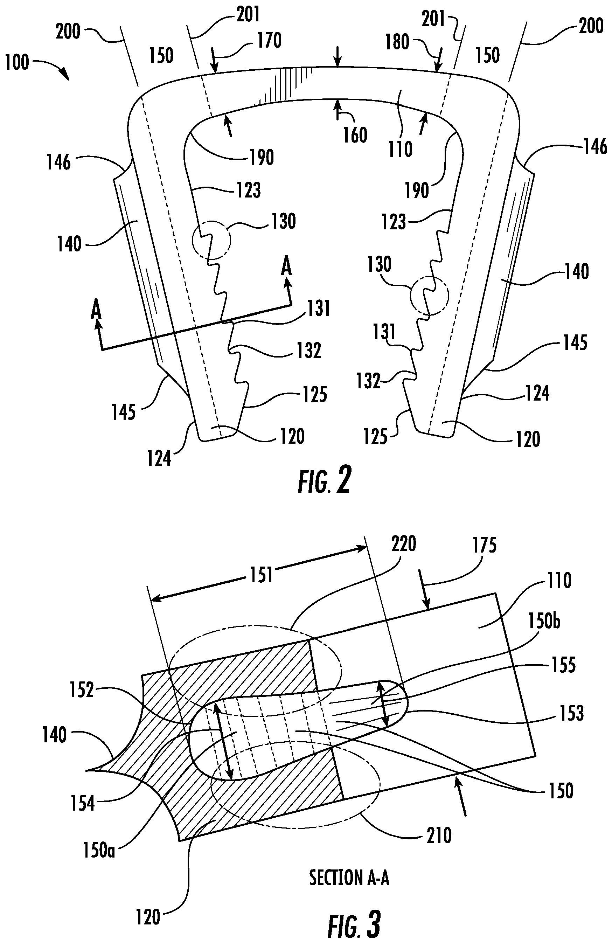

Figure 2 is a front view of implant 100 showing bridge component 110, legs 120, fins 140 and barbs 130. Bridge member 110 is shown with an arched geometry of varying thickness. Bridge member 110 has a center thickness 160 and two outer thicknesses 170 and 180 at the connection region of the bridge 110 to the legs 120. This connection region may be further defined by joint 190 which may be arched, gusseted or some other suitable geometry. In this embodiment, thickness 160 is less than thickness 170 and 180. Thickness 170 and 180 are shown as being equivalent in this particular embodiment. Thickness 160 may be more or less than thickness 170 and thickness 180. Thickness 170 may be more or less than thickness 180. The combination of geometries 160, 170, 180 and 190 are such that the stresses seen by the bridge member 110 and connection region of the bridge member 110 and legs 120 are sufficient to withstand the loading environment of the implant. The legs 120 are shown to have barbs 130. The barbs may have peaks 131 and valleys 132. The barbs 130 may have multiple geometries and configurations. The barbs may or may not be present or needed in a particular embodiment. The merits of the current invention are still viable in the absence of the barbs 130. The number of barbs 130 may vary depending on the length of the leg 120. As shown in this embodiment, the barb peaks 131 are proud to surface 123. The barb valleys 132 are relatively equal to the surface 123. In an alternate embodiment the barb peaks 131 may be below surface 123 or at the level of surface 123. Still further in an alternate embodiment, barb valleys 132 may be below surface 123 or above surface 123. Any combination of the location of the barb peaks 131 and barb valleys 132 relative to surface 123 may be possible. The fin 140 is located on the external leg surface 124. The fin 140 may be proud or extend beyond surface 124. Fin 140 may include a lead-in surface 145 to facilitate insertion into a bone segment. Fin 140 may also include a surface 146 that may sit below a bone surface to prevent or resist pullout of the staple leg 120. Fin 140 may include barbs similar to the barb 130. Fin 140 is of a geometry that creates a non-circular and/or non-symmetrical staple leg such that the geometry of the staple leg 120 will resist rotation within a bone segment. The geometry of the staple leg 120 in combination with the fin 140 and groove 122 may not match the geometry of the prepared hole in the bone for which the staple leg 120 will be inserted. This may create an interference fit between the staple leg 120 and the prepared hole. The implant 100 may be inserted into a bone without preparing a hole in the bone segment. The geometry of the staple leg 120 in combination with the fin 140 and groove 122 may create an interlock between the bone geometry and the implant leg geometry 120 in an unprepared bone segment.

Figure 2 includes dashed lines 200 and 201 to represent opening 150 as also illustrated in Figure 1. Opening 150 is used for attachment to a delivery instrument or inserter. Opening 150 may be used to create groove 122. As shown in Figure 2, opening 150 is positioned such that the opening extends through the top of the bridge 110 to the bottom of the staple legs 120. The opening 150 opens toward the inside of the staple leg 120 creating the groove 122. In an alternate embodiment, groove 122 may open toward the outside of the legs.

Opening 150 in Figure 2 is positioned such that the opening 150 spans the connecting region between bridge 110 and the leg 120 which may include radius 190 thereby opening to the internal region of the staple (i.e. towards the midline of the staple 100). The inner border, 201 of the opening 1 50 may extend into the bridge member 110, The outer border 200 of opening 150 may extend through the entire length of the staple leg 120. The position of the inner border 201 may be advantageous in creating groove 122 and the non-circular, non-symmetrical geometry of the staple leg 120. The position of the inner border 201 may be advantageous in the manufacturing of the implant 100, particularly the geometry of the leg 120, which may include the groove 122. This positioning of the inner border 201 of opening 150 may allow ease of manufacturing by providing a thinner section in the area of thickness 170 or 180 to, for example, provide a starter hole for a wire EDM process or laser cut process or machining process. Embodiments may include the positioning of opening 150.
The positioning of opening 150 may be such that it extends through a thinner section of the implant, in this embodiment the thickness 170 of the implant bridge 110. This may be particularly advantageous in the setup of the manufacturing process. Having the inner border 201 extending through the thinner section 170 may allow the manufacture a simplified more cost effective manufacturing process without having to create groove 122 entirely within the perimeter of the staple leg. The position of opening 150 relative to the staple leg 120 and/or the bridge member 1 10 and/or joint region 190 may provide a means for distributing the required stresses within the implant to achieve a desired compressive force. The position and geometry of opening 150 may be altered to manipulate the stress distribution to achieve the desired performance. The position and geometry of opening 150 may be altered in combination with the thicknesses 170, 160, 180 and/or joint 190 to manipulate the stress distribution to achieve the desired performance. The amount of desired force generated hereby may be accomplished by manipulating individual aspects of the implant geometry and their relative orientations to one another. The geometry and relative orientation of thicknesses 160, 170 and/or 180 may be altered to manipulate the stress distribution within the implant bridge 110 to achieve the desired performance. The geometry and relative orientation of thicknesses 160, 170 and/or 180 may be altered in combination with the joint region 190 geometry and relative orientation to manipulate the stress distribution within the implant bridge 110 and/or implant legs 120 to achieve the desired performance. The geometry and relative orientation of thicknesses 160, 170 and/or 180 may be altered in combination with the joint region 190 geometry and relative orientation and may be in combination with altering the geometry and relative orientation of opening 150 to manipulate the stress distribution within the implant bridge 110 and/or implant legs 120 to achieve the desired performance. The geometry and relative orientation of opening 150 may be altered to manipulate the stress distribution within the implant bridge 110 and/or implant legs 120 to achieve the desired stress distribution and/or performance. Furthermore, the implant width 175 as shown in Figure 3 may also be manipulated in geometry and/or orientation either as an independent variable or in combination with the previously described aspect or aspects of the implant to manipulate the stress distribution within the implant bridge 110 and/or implant legs 120 to achieve the desired performance or force generation.

Figure 3 shows the cross section view A-A as noted in Figure 2 illustrating that the cross section of the opening does not need to be uniform, although a uniform cross section can optionally be used. Opening 150 is further divided into openings 150a and 150b. Opening 150a is represented by the vertical dashed lines in Figure 3 and may be bounded by the portion of opening 150 surrounded by the staple leg 120 and opening toward the midline of the implant 100. This portion 150a of the opening 150 is analogous to the previously discussed groove 122. Opening 150b is represented by the horizontal solid lines in Figure 3 and may be bounded by the portion of opening 150 contained in the staple bridge 110 and inside edge of the staple leg 120. Combined, opening 150a and 150b make up opening 150. Opening 150 may extend out of one particular side of a staple leg or may be contained completely within a staple leg or completely outside a staple leg. Opening 150 has an overall length 151 and may include an outside width 154 and an inside width 155. Widths 154 and 155 may or may not be equal in size. Length 151 is greater than lengths 154 and 155. Width 155 may be more or less than width 154. In this exemplary embodiment opening 150 may have an outer edge generated by a radius 152 and an inner edge generated by radius 153. Radius 152 may or may not be the same as 153. Radius 153 may be greater than or less than 152. The outer borders of opening 150 may or may not be radii. Geometries, such as tear drops, squares, rectangles, ovals, slots, keyholes, etc. may be used to create opening 150. Opening 150 is such that the geometry is more resistant to bending in the direction of the force generated by the implant. In this particular embodiment, space 150 is more resistant to bending in the direction of the compressive force. Space 150 is used to facilitate insertion of the implant or connection to an inserter for imputation. The geometry of space 150 would mimic that geometry of the corresponding insertion tool. The insertion tool would have a geometry similar to that of space 150.

As shown in the exemplary embodiment of Figure 3, the interrupted staple leg geometry 120 may create a geometry that produces multiple fins or features to resist rotation. In this exemplary embodiment fin 140 exists on the outside perimeter of the staple leg 120. In addition other fins may be created in the outer geometry of the staple leg 120 and the groove 122, e.g. space 150a. As shown in Figure 3 the combination of the staple leg geometry 120, the opening 150 and/or groove 122 may be used to make an effective fin 210 and/or an effective fin 220.

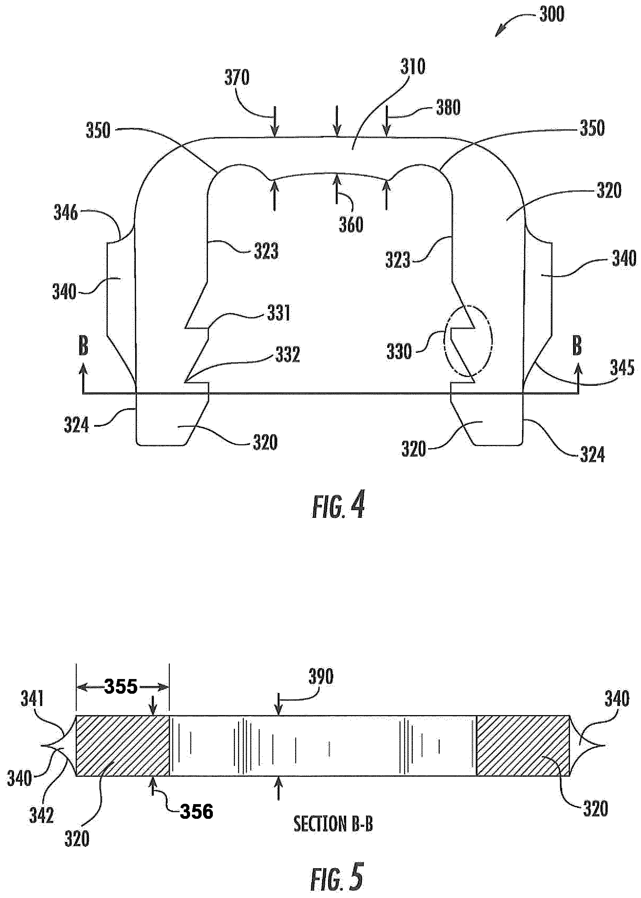

Figure 4 shows an embodiment of the current invention of a staple 300 having a staple bridge 310 and staple legs 320. In this particular embodiment the staple legs 320 are shown parallel relative to each other. Staple bridge 310 may be relatively flat across the top and may have a varying thickness represented by thickness 360, 370 and 380. Bridge member 310 has a center thickness 360 and two outer thicknesses 370 and 380. In this embodiment, thickness 360 is less than thickness 370 and 380. Thicknesses 370 and 380 are shown as being equivalent in this particular embodiment Thickness 360 may be more or less than thickness 370 and thickness 380. Thickness 370 may be more or less than thickness 380. The connection region between the staple leg 320 and the staple bridge 310 has geometry 350. This geometry 350 may be used to facilitate insertion of the implant 300 or connection to an inserter for implantation. This geometry 350 may be arched, gusseted or some other suitable geometry. The combination of geometries of thicknesses 360, 370 and 380 and geometry 350 are such that the stresses seen by the bridge member 310 and connection region of the bridge member 310 and legs 320 are sufficient to withstand the loading environment of the implant. The position of geometry 350 relative to the staple leg 320 and/or the bridge member 310 may provide a means for distributing the required stresses within the implant to achieve a desired compressive force. The position and geometry of 350 may be altered to manipulate the stress distribution to achieve the desired permeance. The position and geometry of undercut 350 may be altered in combination with the thicknesses 370, 360 and/or to manipulate the stress distribution to achieve the desired performance. The amount of desired force generated thereby may be accomplished by manipulating individual aspects of the implant geometry and their relative orientations to one another. The geometry and relative orientation of thicknesses 360, 370 and/or 380 may be altered to manipulate the stress distribution within the implant bridge 310 to achieve the desired performance. The geometry and relative orientation of thicknesses 360, 370 and/or 380 may be altered in combination with the joint region 350 geometry and relative orientation to manipulate the stress distribution within the implant bridge 310 and/or implant legs 320 to achieve the desired performance. The geometry and relative orientation of geometry 350 may be altered to manipulate the stress distribution within the implant bridge 310 and/or implant legs 320 to achieve the desired stress distribution and/or performance. Furthermore, the implant width 390 as shown in Figure 5 may also be manipulated in geometry and/or orientation either as an independent variable or in combination with the previously described aspect or aspects of the implant to manipulate the stress distribution within the implant bridge 310 and/or implant legs 320 to achieve the desired performance or force generation.

The legs 320 are shown to have barbs 330. The barbs may have peaks 331 and valleys 332. The barbs 330 may have multiple geometries and configurations. The barbs may or may not be present or needed in a particular embodiment. The merits of the current invention are still viable in the absence of the barbs 330. The number of barbs 330 may vary depending on the length of the leg 320. As shown in this embodiment, the barb peaks 331 are co-linear with the surface 323. The barb valleys 132 are below the surface 323. In an alternate embodiment the barb peaks 331 may be below surface 323 or above the level of surface 323. Still further in an alternate embodiment, barb valleys 332 may be below surface 323 or above surface 323. Any combination of the location of the barb peaks 331 and barb valleys 332 relative to surface 323 may be possible. The fin 340 is located on the external leg surface 324. The fin 340 may be proud or extend beyond surface 324. Fin 340 may include lead-in surface 345 to facilitate insertion into a bone segment. Fin 340 may also include a surface 346 that may sit below a bone surface to prevent or resist pullout of the staple leg 320. Fin 340 may include barbs similar to the barb 330. Fin 340 is of a geometry that creates a non-circular and/or non-symmetrical staple leg 320 such that the geometry of the staple leg 320 will resist rotation within a bone segment. The geometry of the staple leg 320 in combination with the fin 340 may not match the geometry of the prepared hole in the bone for which the staple leg 320 will be inserted. This may create an interference fit between the staple leg 320 and the prepared hole. The implant 300 may be inserted into a bone without preparing a hole in the bone segment. The geometry of the staple leg 320 in combination with the fin 340 may create an interlock between the bone geometry and the implant leg geometry 320 in an unprepared bone segment.

Figure 5 is the section view B-B as depicted in Figure 4. The staple leg 320 may be of solid construction without a groove. The staple leg 320 may have fin 340 protruding from a surface of the staple leg creating a non-symmetrical and/or non-circular geometry. Staple leg 320 may have a width 355 and a height 356. In this particular embodiment the staple leg height 356 is represented as being relatively equivalent to the staple bridge height 390. In other embodiments, staple leg height 356 may be more or less than staple bridge height 390. Figure 5 represents an embodiment that does not include and opening passing through the staple bridge 310 or the staple leg 320.

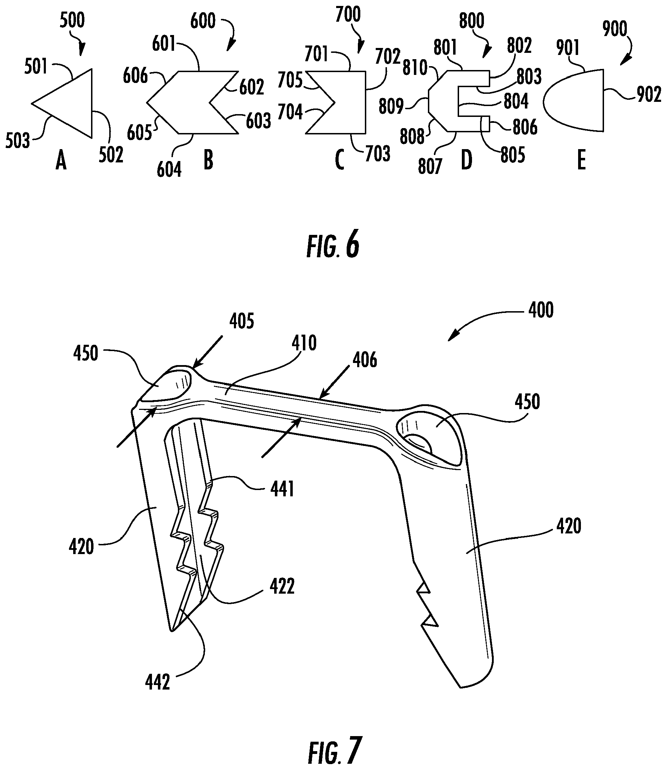

Figure 6 shows other possible cross-section geometries that may be used for staple legs 120 and/or 320. The embodiments shown in Figure 6 have various faces that create a geometry that is non-circular and/or non-symmetrical in at least one plane. The geometry of the staple legs in combination with a fin and/or a groove may not match the geometry of the prepared hole in the bone for which the staple leg will be inserted. This may create an interference fit between the staple leg and the prepared hole. The implant may be inserted into a bone without preparing a receiving hole in the bone segment. The geometry of the staple leg in combination with the fin and/or groove may create an interlock between the bone geometry and the implant leg geometry in an unprepared bone segment. With the current invention, it is also possible to create a broached hole in a bone segment that may match the non-circular and/or non-symmetrical geometry of a staple leg. The combination of the non-circular and/or non-symmetrical leg with a broach of similar geometry would resist rotation of the staple leg in the bone segment.

Figure 7 shows yet another embodiment of the current invention of staple 400 having a bridge 410 and legs 420. In this embodiment the width 405 of the legs 420 is different than the width 406 of the staple bridge 410. Opening 450 is present and extends into the bridge 410. This embodiment shows groove 422 extending the length of the staple leg 420. The combination of the geometry of the staple leg 420, the opening 450, the position of the opening 450 relative to the staple bridge 410 and the groove 422 creates effective fins 441 and 442.

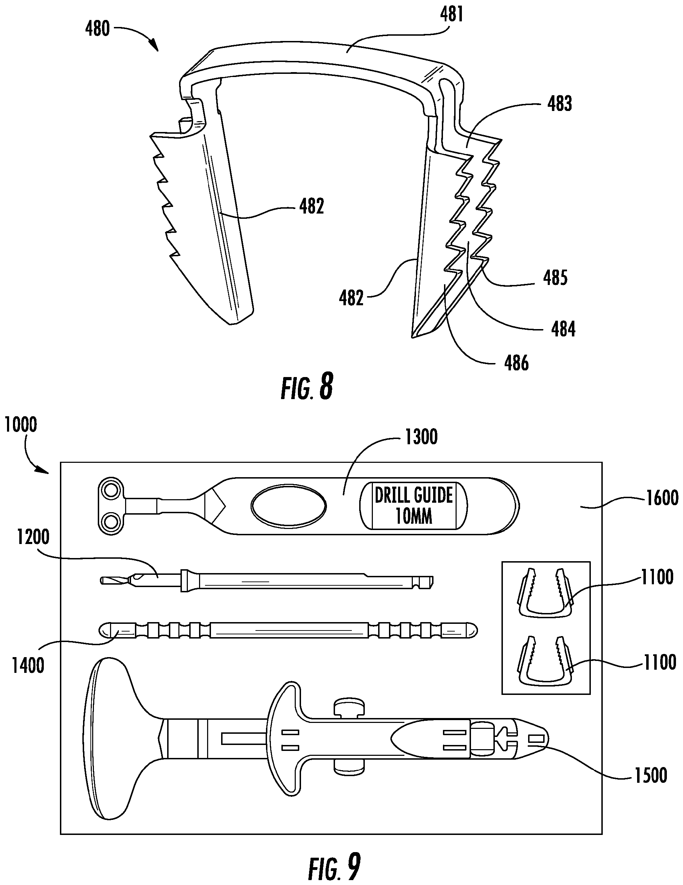

Figure 8 is yet another embodiment of the current invention, staple 480. Staple 480 has a bridge 481 and legs 482. The opening 483 is present but may not extend into the bridge 481. Opening 483 may open to the outside of the staple leg 482 creating groove 484. The combination of the geometry of the staple leg 482, the opening 483, the position of the opening 483 relative to the border of the staple leg and the groove 484 creates effective fins 485 and 486.

The implant of the current invention may be packaged as an implant kit with the associated instruments needed for a successful implantation. Such a kit may include the implant or implants, the necessary drills, reamers or broaches for preparing the bone for receiving the implant, any necessary drill guides and an inserter for facilitating implantation of the implant into the bone. This kit may be provided sterile packaged for single use. Figure 9 shows one embodiment of an implant kit 1000 that may be used to provide the end user with the necessary associated instruments for successfully implanting an implant of the current invention. Such a kit may include one or more implants 1100 similar to the embodiments described herein. The kit may also include a drill 1200, a drill guide 1300 and a provisional fixation pin 1400. The kit may also include an insertion tool or inserter 1500 for facilitating insertion of the implant 1100 into a bone segment. One embodiment of the kit may have the implant 1100 preassembled to the insertion tool 1500. The components of this kit may be assembled in a tray 1600 constructed of appropriate materials. Once the end user opens the kit, the surgical technique may include the following steps. After exposure of the operative site, the osteotomy or fracture may be reduced and held in place. The drill guide or reamer guide may be placed across the fusion site with both guide tubes against the bone. The first hole is drilled to final depth by advancing the drill, reamer or broach until the depth stop hits the top of the guide. A provisional fixation pin may be placed in the prepared hole to help maintain reduction while the second hole is prepared. Once both holes have been prepared, the legs of the implant, which has been assembled or loaded to the inserter tool, may be inserted into the prepared holes in the bone. Since an interference fit may be used, light tapping on the end of the inserter tool may be helpful in advancing the implant into the bone. The implant should be fully inserted until flush against the surface of the bone. The implant may then be released from the inserter tool. Alternatively, the holes in the bone may not be prepared for receiving the legs of the staple. As described herein, certain features of the current invention may be used or beneficial in a technique that does not require preparation of holes in the bone prior to insertion of the implant.

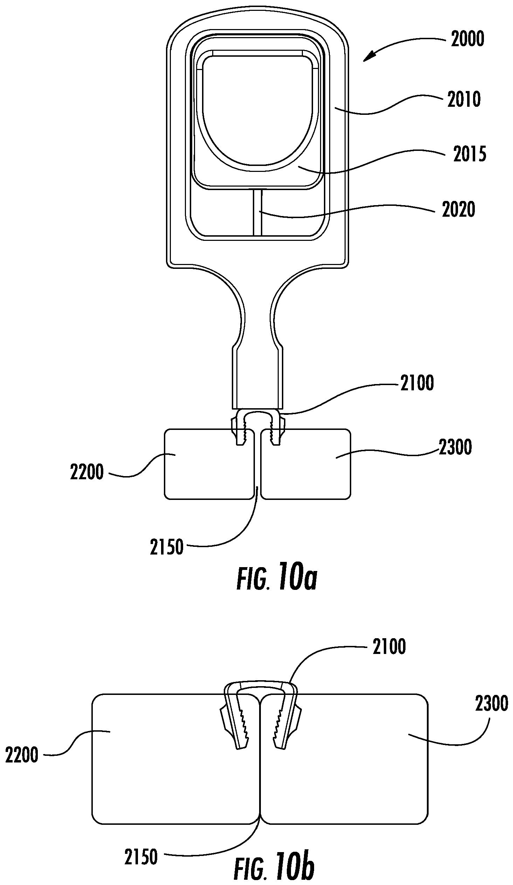

Figure 10a shows an implant 2100 of the current invention assembled to an inserter 2000 of the current invention partially inserted into bones 2200 and 2300. The inserter 2000 may have an inserter body 2010, an inserter plunger 2015 and an ejector pin 2020. The inserter has means for engaging the implant that allow the implant to be positioned flush to bone. For example, this embodiment would have inserter legs of a geometry that would mimic the space 150 as depicted in Figure 3. The inserter legs may be in a fixed orientation. In this embodiment the legs are in a fixed parallel orientation for insertion into the implant 2100. As the inserter legs are placed into the staple 2100, the staple legs are made parallel. The inserter plunger 2015 may push the ejector pin 2020 thereby releasing, ejecting or disengaging the implant 2100 from the inserter body 2010. Figure 10a shows the implant 2100 having parallel legs partially inserted in bones 2200 and 2300 showing the fusion site 2150 having a gap. The legs of implant 2100 are made relatively parallel and are maintained relatively parallel by the inserter 2000 to facilitate insertion into the bones 2200 and 2300. Once the implant 2100 is released from the inserter 2000 as shown in Figure 10b the legs may converge or compress drawing bones 2200 and 2300 toward one another causing the fusion site 2150 to compress with no gap.

The previous description of the embodiments described herein may be manufactured from a number of materials including those with elastic properties, such as super elastic nitinol. However this description is not intended to be limited by the materials of construction. Those skilled in the art will understand, based on the description of the invention herein, that the same may be accomplished using a material for example with shape memory aspects, such as shape memory nitinol or other materials that currently exist that have desirable material properties that will achieve the intended function of the current invention, for example stainless steel and/or titanium or titanium alloys. The use of an inserter/holding device may or may not be optional. The specific details of the inserter may vary greatly depending of the chosen embodiment. The exemplary embodiments described herein describe a one-piece staple-like implant. Those skilled in the art will understand, based on the description of the disclosure herein that an implant of multiple components would still be within the disclosure. Other embodiments may include two or more fixation means that may be of the same style (such as bone screws, bone pegs, blades, staple legs, etc.) or of varying styles or some combination thereof. For fixation means that are modular in nature, they may or may not be made of the same material as the implant described herein. The embodiments described herein contemplate that both staple legs will include the same features. However, staple legs within the same implant may vary in their geometry, fins, length, cross section, barbs, or other physical characteristics relative to each other. Furthermore, one or more of the staple legs may be of a circular or symmetrical geometry while one or more of the other staple legs may be within this disclosure.

The exemplary embodiments described herein are not intended to be limiting. The embodiments described herein can be manufactured from a number of different materials or combinations of materials. In the exemplary embodiments described herein, the implant may be made of a material that may have elastic or spring properties or shape memory properties that may allow the implant to have more than one configuration. Nitinol, for example, possesses material properties, such as shape memory and/or super elasticity that may provide the inherent properties to allow an embodiment to have multiple configurations. Still other materials such as PEEK or other polymers may also possess material properties beneficial for the embodiments as described herein. A combination of materials may also be preferred. The disclosure would apply to staples or implants manufactured from a number of materials such as nitinol, stainless steel, titanium, PEEK, polymers, biologic or resorbable materials. Based on the description of the invention herein, those skilled in the art will realize the merits of the current invention are independent of material but may be more beneficial in some materials than others. For example, certain examples of the invention may be more appealing in nitinol given its dynamic compressive abilities in bone staples and its difficulty in manufacturability. Based on the description of the invention herein, those skilled in the art will realize the benefits of the current invention.

The scope of the invention is defined by the claims.

## Claims

1. An assembly for delivering an implant to a surgical site for osteosynthesis comprising:
a delivery instrument (2000); and
an implant (100, 2100) having a first configuration when it is releasably attached to a delivery instrument and a second configuration when it is not attached to a delivery instrument, the implant or a portion thereof being comprised of a material that allows the implant to have multiple configurations,
the implant having non-symmetrical means for fixation to bone segments (120), means to prevent or minimize rotation of the bone segments relative to one another (140) and means to resist pullout (130) of the implant from the bone segments;
**characterized in that** the means to prevent or minimize rotation of the bone segments relative to one another comprises a fin (140), wherein the fin is of a geometry that creates the non-symmetrical geometry of the means for fixation to bone segments.

2. The assembly of claim 1 wherein the implant further comprises means for releasably engaging the delivery instrument.

3. The assembly of claim 1 wherein the implant has two means for fixation to bone segments wherein the second configuration causes the means for fixation to compress and/or distract and/or control spatial orientation of the bone segments relative to one another.

4. The assembly of claim 1 wherein the non-symmetrical means for fixation to bone segments (120) are leg members (120) and wherein the fin (140) is located on an external surface of one of the leg members.

5. The assembly of claim 4 wherein the leg members (120) comprise means for releasably engaging the delivery instrument.

6. The assembly of claim 5 wherein the means for releasably engaging the delivery instrument comprise non-circular elongated openings (150) extending through the legs (120).

7. The assembly implant of claim 6 wherein:
the non-circular openings (150) are in the shape of a tear drop, square, rectangle, oval, slot or keyhole; or
wherein the leg members (120) are of uniform thickness; or
wherein the leg members are not of uniform thickness; or
wherein the non-circular elongated openings (150) are of uniform cross section along the lengths thereof; or
wherein the non-circular elongated openings (150) are not of uniform cross section along the lengths thereof.

8. The assembly implant of claim 4 wherein the leg members (120) have tapered tips configured to facilitate insertion into bone; or
wherein the leg members (120) are connected by a bridge member (110) and the bridge member is of uniform thickness; or
wherein the leg members (120) are connected by a bridge member (110) and the bridge member is not of uniform thickness; or
wherein the leg members (120) have a uniform cross section along the lengths thereof; or
wherein the leg members (120) do not have a uniform cross section along the lengths thereof.

9. The assembly of claim 1 wherein the delivery instrument (2000) has a fixed engagement means for releasably engaging the implant;
optionally wherein the fixed engagement means is comprised of parallel legs which force the implant into the first configuration.

10. The assembly of claim 1 wherein the delivery instrument (2000) has a means for ejecting the implant;
optionally wherein the means for ejecting is an ejector pin (2020).

11. The assembly of claim 1 wherein the delivery instrument (2000) has a connection means with the implant, the connection means being elongated to maintain the implant in the first configuration for insertion into bone or other tissue.

12. An implant (100, 2100) as defined in any of claims 1-8.

13. A kit comprising the implant of claim 12 and further comprising drills, drill guides, reamers, broaches and/or a delivery instrument.

14. The kit of claim 13 comprising a sterile package.

15. The kit of claim 13 wherein the implant (100, 2100) is releasably attached to the delivery instrument (2000); or
wherein the implant is releasably attached to a holding apparatus for attaching to an inserter assembly.

## Patentansprüche

1. Anordnung zum Abgeben eines Implantats an eine Operationsstelle zur Osteosynthese, umfassend:
ein Abgabeinstrument (2000); und
ein Implantat (100, 2100) mit einer ersten Konfiguration, wenn es lösbar an ein Abgabeinstrument befestigt ist, und einer zweiten Konfiguration, wenn es nicht an ein Abgabeinstrument befestigt ist, wobei das Implantat oder ein Teil davon aus einem Material besteht, das dem Implantat ermöglicht, mehrere Konfigurationen zu haben, wobei das Implantat zur Befestigung an Knochensegmente (120) nicht-symmetrische Mittel, Mittel zum Verhindern oder Minimieren der Drehung der Knochensegmente relativ zueinander (140) und Mittel zum Widerstand gegenüber dem Herausziehen (130) des Implantats aus den Knochensegmenten aufweist;
**dadurch gekennzeichnet, dass** die Mittel zum Verhindern oder Minimieren der Drehung der Knochensegmente relativ zueinander eine Lamelle (140) umfassen, wobei die Lamelle eine Geometrie aufweist, die die nicht-symmetrische Geometrie der Mittel zum Befestigen an Knochensegmente schafft.

2. Anordnung nach Anspruch 1, wobei das Implantat ferner Mittel zum lösbaren Ineingriffnehmen des Abgabeinstruments umfasst.

3. Anordnung nach Anspruch 1, wobei das Implantant zwei Mittel zum Befestigen an Knochensegmente aufweist, wobei die zweite Konfiguration die Mittel zum Befestigen zum Komprimieren und/oder Ablenken und/oder Steuern der räumlichen Ausrichtung der Knochensegmente relativ zueinander veranlasst.

4. Anordnung nach Anspruch 1, wobei das nicht-symmetrische Mittel zum Befestigen an Knochensegmente (120) Beinelemente (120) beinhaltet, und wobei die Lamelle (140) an einer externen Fläche des einen der Beinelemente angeordnet ist.

5. Anordnung nach Anspruch 4, wobei die Beinelemente (120) Mittel zum lösbaren Ineingriffnehmen des Abgabeinstruments umfassen.

6. Anordnung nach Anspruch 5, wobei die Mittel zum lösbaren Ineingriffnehmen des Abgabeinstruments nicht-kreisförmige längliche Öffnungen (150) umfassen, die sich durch die Beine (120) erstrecken.

7. Anordnungsimplantat nach Anspruch 6, wobei:
die nicht-kreisförmigen Öffnungen (150) in Form eines Tropfens, Quadrats, Rechtecks, Ovals, Schlitzes oder Schlüssellochs sind; oder
wobei die Beinelemente (120) eine gleichförmige Dicke aufweisen; oder
wobei die Beinelemente keine gleichförmige Dicke aufweisen; oder
wobei die nicht-kreisförmigen länglichen Öffnungen (150) entlang der Längen davon einen gleichförmigen Querschnitt aufweisen; oder
wobei die nicht-kreisförmigen länglichen Öffnungen (150) entlang der Längen davon keinen gleichförmigen Querschnitt aufweisen.

8. Anordnungsimplantat nach Anspruch 4, wobei die Beinelemente (120) sich verjüngende Spitzen aufweisen, die konfiguriert sind, um die Einführung in Knochen zu erleichtern; oder
wobei die Beinelemente (120) durch ein Brückenelement (110) verbunden sind und das Brückenelement eine gleichförmige Dicke aufweist; oder
wobei die Beinelemente (120) durch ein Brückenelement (110) verbunden sind und das Brückenelement keine gleichförmige Dicke aufweist; oder
wobei die Beinelemente (120) entlang der Längen davon einen gleichförmigen Querschnitt aufweisen; oder
wobei die Beinelemente (120) entlang der Längen davon keinen gleichförmigen Querschnitt aufweisen.

9. Anordnung nach Anspruch 1, wobei das Abgabeinstrument (2000) ein festes Eingriffsmittel zum lösbaren Ineingriffnehmen des Implantats aufweist;
optional wobei das feste Eingriffsmittel aus parallelen Beinen besteht, die das Implantant in die erste Konfiguration zwingen.

10. Anordnung nach Anspruch 1, wobei das Abgabeinstrument (2000) ein Mittel zum Ausstoßen des Implantats aufweist;
Optional wobei das Mittel zum Ausstoßen ein Ausstoßstift (2020) ist.

11. Anordnung nach Anspruch 1, wobei das Abgabeinstrument (2000) ein Verbindungsmittel mit dem Implantat aufweist, wobei das Verbindungsmittel länglich ist, um das Implantat in der ersten Konfiguration zur Einführung in Knochen oder anderes Gewebe zu halten.

12. Implantat (100, 2100) nach einem der Ansprüche 1 bis 8.

13. Set, umfassend das Implantat nach Anspruch 12 und ferner umfassend Bohrer, Bohrerführungen, Fräser, Räumer und/oder ein Abgabeinstrument.

14. Set nach Anspruch 13, ferner umfassend eine sterile Verpackung.

15. Set nach Anspruch 13, wobei das Implantat (100, 2100) lösbar an das Abgabeinstrument (2000) befestigt ist; oder
wobei das Implantat lösbar an eine Haltevorrichtung zum Anbringen an eine Einführeranordnung befestigt ist.

## Revendications

1. Ensemble permettant de livrer un implant sur un site chirurgical destiné à l'ostéosynthèse comprenant :
un instrument de livraison (2000) ; et
un implant (100, 2100) ayant une première configuration lorsqu'il est fixé de manière amovible à un instrument de livraison et une seconde configuration lorsqu'il n'est pas attaché à un instrument de livraison, l'implant ou une partie de celui-ci étant constitué d'un matériau qui permet à l'implant d'avoir de multiples configurations,
l'implant comportant un moyen non symétrique d'attache à des segments osseux (120), un moyen permettant d'empêcher ou de minimiser la rotation des segments osseux les uns par rapport aux autres (140) et un moyen de résister au retrait (130) de l'implant des segments osseux ;
**caractérisé en ce que** le moyen permettant d'empêcher ou de minimiser la rotation des segments osseux les uns par rapport aux autres comprend un aileron (140), l'aileron étant d'une géométrie qui créé la géométrie non symétrique du moyen d'attache aux segments osseux.

2. Ensemble selon la revendication 1, dans lequel l'implant comprend en outre un moyen permettant de mettre en prise l'instrument de livraison de manière amovible.

3. Ensemble selon la revendication 1, dans lequel l'implant comporte deux moyens d'attache aux segments osseux, dans lequel la seconde configuration amène le moyen d'attache à comprimer et/ou à distraire et/ou à commander l'orientation spatiale des segments osseux les uns par rapport aux autres.

4. Ensemble selon la revendication 1, dans lequel le moyen d'attache non symétrique aux segments osseux (120) sont des éléments pieds (120) et dans lequel l'aileron (140) est situé sur une surface externe d'un des éléments pieds.

5. Ensemble selon la revendication 4, dans lequel les éléments pieds (120) comprennent un moyen permettant de mettre en prise l'instrument de livraison de manière amovible.

6. Ensemble selon la revendication 5, dans lequel le moyen permettant de mettre en prise l'instrument de livraison de manière amovible comprend des ouvertures allongées non circulaires (150) s'étendant à travers les pattes (120).

7. Ensemble implant selon la revendication 6 dans lequel :
les ouvertures non circulaires (150) sont en forme de larme, carrée, rectangle, ovale, de fente ou de trou de serrure ; ou
dans lequel les éléments pieds (120) ont une épaisseur uniforme ; ou
dans lequel les éléments pieds ne sont pas d'épaisseur uniforme ; ou
dans lequel les ouvertures allongées non circulaires (150) ont une section transversale uniforme le long de leur longueur ; ou
dans lequel les ouvertures allongées non circulaires (150) n'ont pas de section transversale uniforme le long de leur longueur.

8. Ensemble implant selon la revendication 4 dans lequel les éléments pieds (120) des pointes coniques conçues pour faciliter l'insertion dans l'os ; ou
dans lequel les éléments pieds (120) sont reliés au moyen d'un élément de (110) et l'élément de pont est d'épaisseur uniforme ; ou
dans lequel les éléments pieds (120) sont reliées au moyen d'un élément (110) et l'élément de pont n'est pas d'épaisseur uniforme ; ou
dans lequel les éléments pieds (120) ont une section transversale uniforme le long de leur longueur ; ou
dans lequel les éléments pieds (120) n'ont pas de section transversale uniforme le long de leur longueur.

9. Ensemble selon la revendication 1 dans lequel l'instrument de livraison (2000) comporte un moyen de contact fixe permettant de mettre en prise l'implant de manière amovible ;
dans lequel le moyen de contact fixe est éventuellement constitué de pattes parallèles qui forcent l'implant dans la première configuration.

10. Ensemble selon la revendication 1 dans lequel l'instrument de livraison (2000) comporte un moyen d'éjection de l'implant ;
dans lequel le moyen d'éjection est éventuellement une broche d'éjection (2020).

11. Ensemble selon la revendication 1 dans lequel l'instrument de livraison (2000) comprend un moyen de raccordement avec l'implant, le moyen de raccordement étant allongé afin de maintenir l'implant dans la première configuration pour une insertion sur des os ou d'autres tissus.

12. Implant (100, 2100) selon l'une quelconque des revendications 1 à 8.

13. Kit comprenant l'implant selon la revendication 12 et comprenant en outre des forets, des guides de foret, des alésoirs, des broches et/ou un instrument de livraison.

14. Kit selon la revendication 13 comprenant un emballage stérile.

15. Kit selon la revendication 13 dans lequel l'implant (100, 2100) est fixé de manière amovible à l'instrument de livraison (2000) ; ou
dans lequel l'implant est fixé de manière amovible à un appareil de maintien permettant de se fixer à un ensemble d'insertion.
